# EUROPÄISCHE PATENTANMELDUNG

(11) **EP 3 051 289 A1**
(43) Veröffentlichungstag der Anmeldung: **03.08.2016**
(21) Anmeldenummer: 15000226.9
(22) Anmeldetag: 27.01.2015
(51) Int. Cl.: G01N 33/28

(54) **Apparatur und Verfahren zur automatisierten Oktanzahlbestimmung von Ottokraftstoffen**

(71) Anmelder: ASG Analytik-Service Gesellschaft mbH, 86356 Neusäss (DE)
(72) Erfinder: Wilharm, Thomas, 86497 Horgau (DE); Seidenspinner, Philipp, 80805 München (DE)
(74) Vertreter: Gallo, Wolfgang

(57) **Zusammenfassung**

Apparatur und Verfahren zur Oktanzahlbestimmung von Ottokraftstoffen durch Einspritzen des Kraftstoffs in eine temperierte Konstantvolumen-Brennkammer und Aufnahme des Druckverlaufs oder Lichtverlaufs in der Brennkammer nach Selbst- oder Fremdzündung des Kraftstoffs, und Bestimmen der Oktanzahl aus dem Zündverzug aufgrund des Druckverlaufs oder Lichtverlaufs.

## Beschreibung

Die Erfindung betrifft eine Apparatur und ein Verfahren zur Oktanzahlbestimmung von Ottokraftstoffen und Raffinerieströmen.

Die Oktanzahl ist bekanntlich ein Maß für die Klopffestigkeit von Ottokraftstoffen.

Beispiele für Ottokraftstoffe sind Raffinerieprodukte mit einem Siedebereich von 25 °C bis 210 °C, die als Normal- und Superbenzin, Reformulate oder aus biogenen Komponenten erzeugt erhältlich sind.

Die Oktanzahl wird in der Norm EN ISO 5163 für die MOZ (Motor-Oktan-Zahl) und in der Norm EN ISO 5164 für die ROZ (Research-Oktan-Zahl) definiert, und dort wird auch deren Bestimmung in Prüfmotoren beschrieben.

Die Bestimmung von Oktanzahlen erfolgt üblicherweise unter Verwendung eines genormten Viertakt-Einzylinder-Motors mit variablem Verdichtungsverhältnis und Vergaser. Dieses bekannte motorische Verfahren zur Oktanzahlbestimmung ist allerdings aufwendig, zeitraubend und damit teuer, denn die Nachteile der Bestimmung der Oktanzahl mit einem Prüfmotor sind hoher Kraftstoffverbrauch, wartungsintensiver Betrieb der Prüfeinrichtung, und lange Versuchsdauern.

Ein solches Verfahren unter Verwendung eines Prüfmotors ist auch in der WO 2009/130254 beschrieben. Dort wird vorgeschlagen, den zeitlichen Verlauf des Zylinderdrucks des Prüfmotors während der Verbrennung des Kraftstoffs im Prüfmotor zu ermitteln und die ermittelten Drucksignale mit den entsprechenden Drucksignalen wenigstens eines Norm-Kraftstoffs bekannter Klopffestigkeit zu vergleichen. Ein ähnliches Verfahren ist auch aus der EP 0 039 881 bekannt.

Demgegenüber liegt der Erfindung die Aufgabe zugrunde, eine Apparatur und ein Verfahren zu schaffen, mit welcher eine wesentlich einfachere und schnellere, weniger aufwendige und obendrein automatisierte Oktanzahlbestimmung von Ottokraftstoffen möglich ist.

Diese Aufgabe wird gemäß der Erfindung durch die im unabhängigen Vorrichtungsanspruch angegebene Apparatur und das im unabhängigen Verfahrensanspruch angegebene Verfahren gelöst. Vorteilhafte Ausgestaltungen und Weiterbildungen der Apparatur sind Gegenstand der Unteransprüche.

Erfindungsgemäß wird mit einer Konstantvolumen-Apparatur zur Oktanzahlbestimmung gearbeitet, und ein Messzyklus zur Bestimmung der Oktanzahl umfasst folgende Arbeitsschritte:
(1) Befüllung einer Konstantvolumen-Brennkammer mit Druckluft, und Aufheizung der Druckluft in der Brennkammer auf eine vorgegebene Temperatur;
(2) Kraftstoffeinspritzung in die Brennkammer mittels einer Einspritzdüse;
(3) Zünden des Kraftstoffs in der Brennkammer durch Selbstzündung oder Fremdzündung durch eine Zündkerze, einen Glühstift oder einer Lasereinrichtung;
(4) Messaufnahme des Druckverlaufs in der Brennkammer infolge der Selbst- oder Fremdzündung des Kraftstoffs und dessen Verbrennung;
(5) Berechnung der Oktanzahl aus dem gemessenen Zündverzug.

Die Zeit zwischen der Kraftstoffeinspritzung und der Kraftstoffzündung, d. h. der Zündverzug, wird möglichst genau gemessen. Dieser Zündverzug beträgt bei konventionellen Ottokraftstoffen in einer Konstantvolumen-Apparatur typischerweise etwa 3 ms bis 25 ms. Durch Messung von Referenzkraftstoffen oder primären Standards (Mischungen aus n-Heptan, Isooktan und Toluol) mit bekannter Oktanzahl unter gleichen Bedingungen kann die Apparatur kalibriert werden.

Die Erfassung des Zündzeitpunkts, also des Zeitpunkts des Verbrennungsbeginns, kann entweder durch Erfassung des Druckverlaufs in der Brennkammer mittels eines Drucksensors, oder durch optische Erfassung des Lichts beim Entflammen des Kraftstoff-Luftgemischs mittels einer optischen Sonde erfolgen.

Mit der Erfindung wird eine automatisierte Oktanzahlbestimmung mit hoher Genauigkeit und einem Höchstmaß an Reproduzierbarkeit erreicht, die das bei den bekannten Verfahren unter Verwendung eines Prüfmotors nicht möglich ist.

Ein Ausführungsbeispiel der Erfindung wird nachstehend unter Bezugnahme auf die anliegende schematische Zeichnung in näheren Einzelheiten beschrieben, die in Gestalt eines Blockdiagramms eine Apparatur nach der Erfindung zeigt.

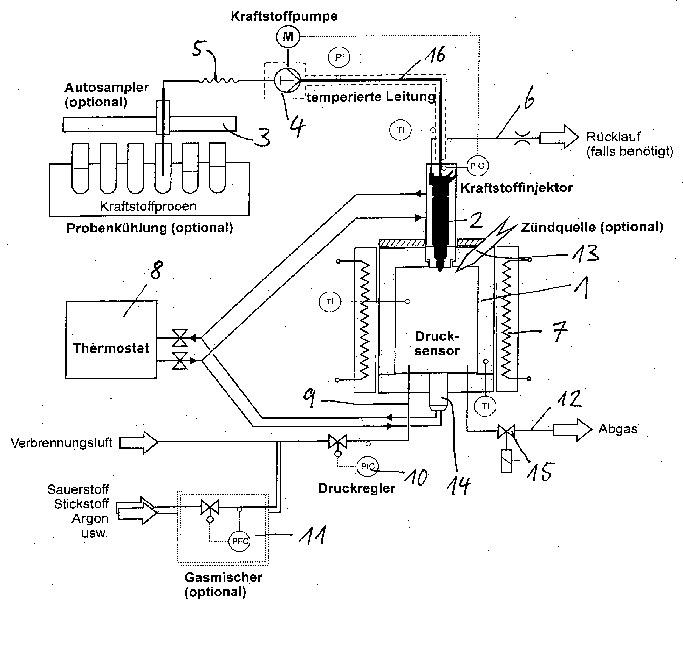

Die in der Zeichnung dargestellte Apparatur umfasst als Kernbestandteil eine Konstantvolumen-Brennkammer 1, in welcher der zu untersuchende Kraftstoff verbrannt wird. Der Konstantvolumen-Brennkammer 1 ist ein Kraftstoffinjektor 2 zum Einspritzen von Kraftstoff zugeordnet. Dieser kann ein handelsüblicher piezoelektrischer Injektor oder ein Mägnetinjektor für Dieselkraftstoffe oder ein Ottokraftstoffinjektor mit einer Ein- oder Mehrlochdüse für den Einsatz in Ottomotoren sein. Der Injektor 2 zerstäubt den Kraftstoff unter Hochdruck in die Brennkammer 1. Der Einspritzdruck beträgt je nach gewählten Versuchsbedingungen zwischen 200 bar und 1.200 bar. Mittels solcher Injektoren ist es möglich, zu einem genau bestimmten Zeitpunkt über eine definierte Zeitdauer eine genau definierte Kraftstoffmenge unter hohem Druck zu zerstäuben.

Der Kraftstoff wird dem Kraftstoffinjektor 2 von einem Probengeber 3 für eine automatisierte Probenzuführung über eine Kraftstoffpumpe 4 zugeführt. Die automatisierte Probenzuführung aus dem Probengeber 3 erfolgt, indem jeweils Kraftstoff aus einem bestimmten Probengefäß aus einer Vielzahl vorhandener Probengefäße im Probengeber 3 mittels der Kraftstoffpumpe 4 angesaugt wird. Solche automatisierten Pröbenzuführungen sind als sogenannte Autosampler bekannt. Der Probengeber 3 kann optional mit einer Probenkühlung versehen sein.

Alternativ kann auch ein elektrisch oder pneumatisch ansteuerbares Schaltventil verwendet werden. In diesem Fall ist die Ansaugleitung der Kraftstoffpumpe über das Schaltventil gleichzeitig an mehrere Probengefäße oder an eine Produktleitung angeschlossen, und über das Schaltventil kann dann eine bestimmte Probe angesaugt werden.

Zwischen dem Probengeber 3 und der Kraftstoffpumpe 4 kann ein Filter 5 angeordnet sein.

Die Kraftstoffleitung 16 von der Kraftstoffpumpe 4 zum Kraftstoffinjektor 2 ist temperiert, was mittels einer der Kraftstoffleitung zugeordneten elektrischen Widerstandsheizung erfolgen kann, um den Kraftstoff mit genau bestimmter Temperatur durch den Kraftstoffin-jektor 2 in die Brennkammer 1 einzuspritzen. Denn für die Wiederholgenauigkeit der Oktanzahlbestimmung ist es wichtig, dass der Kraftstoff im Kraftstoffinjektor möglichst bis in die Düsenspitze eine konstante und definierte Temperatur aufweist. Dadurch wird sichergestellt, dass eine Kraftstoffprobe bei wiederholter Einspritzung eine stets gleichbleibende Viskosität und Dichte hat.

Der Kraftstoffinjektor 2 ist daher im unteren, in der Brennkammerwand angeordneten Teil vorzugsweise mit einem bis dicht an die Ein- oder Mehrlochdüsen reichenden selbsttragenden Kühlmantel ummantelt, der in seinem Inneren Bohrungen und Hohlraume enthält, die mit thermostatisierter Kühlflüssigkeit, bevorzugt Wasser, druckbeaufschlagt durchflossen werden. Durch diesen Kühlmantel kann die Einspritzdüse des Kraftstoffinjektors 2 trotz heißer Brennkammer 1 auf einer definierten Temperatur gehalten werden. Der Kühlmantel ist mechanisch lösbar mit der Brennkammer 1 verbunden, und der Kraftstoffinjektor ist mit dem Kühlmantel lösbar verbunden. Die Temperierung der Kühlflüssigkeit erfolgt mittels eines Thermostaten 8.

Von der Kraftstoffleitung 16 zwischen der Kraftstoffpumpe 4 und dem Kraftstoffinjektor 2 kann, falls benötigt, eine Rücklaufleitung 6 abzweigen. Diese ist auch zweckmäßig, um ein Spülen der Apparatur mit einer neuen Kraftstoffprobe vor dem Einspritzen in die Brennkammer zu ermöglichen, damit sichergestellt ist, dass keine Verunreinigung mit Resten einer vorherigen Kraftstoffprobe vorhanden ist.

Die Brennkammer 1 ist ebenfalls temperiert, um definierte Messkonditionen zu erzeugen. Dazu ist die Brennkammer 1 mit einem Heizmantel 7 versehen, deren Heizung vorzugsweise als elektrische Widerstandsheizung ausgebildet ist und der zur Minimierung von Wärmeverlusten der Apparatur von einer äußeren Wärmeisolation umgeben ist. Die Temperierung der Brennkammer 1 sorgt dafür, dass die Lufttemperatur im Brennkammerinnenraum auf eine vörbestimmte feste Temperatur mit geringer Toleranz eingestellt werden kann.

Die Temperierung der Kraftstoffleitung zwischen der Kraftstoffpumpe 4 und dem Kraftstoffinjektor 2, sowie die Temperierung des Kraftstoffinjektors 2 selbst, als auch die Temperierung der Brennkammer 1 erfolgt rechnergesteuert.

Der Innenraum der Brennkammer 1 ist über eine Luftleitung 9 mit druckbeaufschlagter Verbrennungsluft befüllbar, deren Druck durch einen Druckregler 10 gesteuert wird. Der Verbrennungsluft können optional über einen Gasmischer Sauerstoff, Stickstoff, Argon usw. nach Bedarf zugemischt werden, um eine gewünschte Verbrennungsluftzusammensetzung zu erreichen.

Das Abgas aus der Brennkammer 1 kann durch eine über ein Abgasventil 15 gesteuerte Abgasleitung 12 aus der Brennkammer 1 austreten.

Der Brennkammer 1 ist außerdem optional eine Zündquelle 13 zur Fremdzündung des eingespritzten Kraftstoffs zugeordnet.

Schließlich ist der Brennkammer 1 ein Drucksensor 14 zugeordnet, der als hochdynamischer Quarz-Druckaufnehmer zur Registrierung schneller Druckanstiege ausgebildet sein kann. Dieser Drucksensor kann wahlweise brennraumbündig oder zurückversetzt mit dem Brennkammerinnenraum verbunden sein. Der Drucksensor dient zur Erfassung des Druckverlaufs in der Brennkammer und damit zur Bestimmung des Zeitpunkts der Kraftstoffzündung und des Zündverzugs.

Alternativ kann ein optischer Sensor, beispielsweise eine Glasfasersonde, vorgesehen sein, um den Zündzeitpunkt zu erfassen, indem das beim Entflammen des Kraftstoff-Luft-Gemischs erzeugte Licht ausgewertet wird.

Alle Arbeits- und Messschritte eines Messzyklus durch die eben beschriebene Apparatur werden mittels Software durch einen Rechner (nicht dargestellt) gesteuert und laufen automatisiert ab.

Beim Befüllen der Brennkammer 1 mit Druckluft erfolgt die Druckregelung mittels des autonom arbeitenden Druckreglers 10, der vom Rechner den Sollwert erhält und den IstWert an den Rechner zurück übermittelt.

Die automatisierte Bestimmung der Oktanzahl mittels der beschriebenen Apparatur erfolgt mit folgenden Verfahrensschritten:
S1: Auswählen einer Kraftstoffprobe: Im Rechner ist eine Liste aller Kraftstoffproben und deren Lagerplätze im Probengeber 3 gespeichert. Programmgesteuert wird eine Probe ausgewählt und die Ansaugleitung der Kraftstoffpumpe 4 rechnergesteuert mit dem Lagerplatz dieser Probe verbunden.
S2: Befüllung der Apparatur mit Kraftstoff: Die Kraftstoffpumpe 4 saugt die gewählte Probe an und fördert sie so lange, bis das Leitungsvolumen gespült ist. Dabei kann auch die Einspritzdüse des Kraftstoffinjektors 2 durchgespült werden.
S3a: Kraftstoff-Druckaufbau: Über die Kraftstoffpumpe 4 wird der Druck in der Kraftstoffleitung rechnergesteuert auf den Einspritzdruck-Sollwert aufgebaut.
S3b: Luftdruckeinstellung in der Brennkammer: Über den Druckregler 10 wird rechnergesteuert ein gewünschter definierter Luftdruck im Brennkammerinnenraum erzeugt.
S3c: Einstellung der Verbrennungsluft-Temperatur: Die Verbrennungsluft im Brennkammerinnenraum wird durch die temperierte Brennkammer 1 beheizt, bis die gewünschte definierte Verbrennungsluft-Temperatur im Brennkammerinnenraum erreicht ist.

Die Schritte S3a bis S3c können sequentiell oder zeitlich parallel zueinander stattfinden.
S4: Einspritzen und Zünden des Kraftstoffs: Der Rechner (nicht dargestellt) überprüft durch Abfrage der autonom arbeitenden Regelorgane (Kraftstoffpumpe 4, Temperaturregler 8, Druckregler 10) und/oder Messwerterfassung über entsprechende Messfühler, ob alle Soll-Werte innerhalb vordefinierter Grenzen eingehalten werden, nämlich Kraftstoffdruck, Luftdruck in der Brennkammer, und Lufttemperatur in der Brennkammer. Haben alle diese Parameter die vorgeschriebenen Werte erreicht, löst der Rechner den Kraftstoffeinspritzvorgang durch den Kraftstoffinjektor 2 aus. Dabei wird ein definiertes Strom- oder Spannungssignal so an die als Injektor 2 ausgebildete Einspritzdüse geschickt, dass die Injektorbestromungsdauer den vorgegebenen Wert erreicht. Der zeitliche Verlauf ab Beginn der Injektorbestromung wird durch den Rechner aufgezeichnet. Die Zündung des Kraftstoffs erfolgt durch Selbstzündung oder optional durch rechnergesteuerte Betätigung der Zündquelle 13, wobei im letzteren Fall der Rechner den Zeitpunkt der Zündung durch die Zündquelle mit aufzeichnet.
S5: Erfassung des Druckverlaufs in der Brennkammer: Gleichzeitig mit dem zeitlichen Beginn der Injektorbestromung registriert der Rechner über den Drucksensor 14 den zeitlichen Druckverlauf (oder über einen optischen Sensor den Lichtverlauf) im Brennkammerinnenraum. Die Zeitdifferenz zwischen Injektorbestromung oder Zündung durch die Zündquelle 13 und dem Beginn des Druckanstiegs (oder des Flammenlichts) im Brennkammerinnenraum wird als Zündverzugszeit zur Berechnung der Oktanzahl herangezogen.
S6: Abblasen des Abgases: Der Rechner unterbricht über den Druckregler 10 die Verbrennungsluftzufuhr zum Brennkammerinnenraum und öffnet das Abgasventil 15 in der Abgasleitung 12.

Die Schritte S3a bis S6 werden wenigstens einmal wiederholt. Aus den erhaltenen Einzelergebnissen wird dann eine mittlere Oktanzahl für eine bestimmte Kraftstöffprobe berechnet. Danach wird, beginnend mit der Auswahl einer neuen Kraftstoffprobe, der beschriebene Verfahrensablauf wiederholt.

## Patentansprüche

1. Apparatur zur automatisierten Oktanzahlbestimmung von Kraftstoffen,
**gekennzeichnet durch**
eine Konstantvolumen-Brennkammer (1),
eine dieser zugeordnete Kraftstoffeinspritzdüse (2) sowie Mittel (4) zur Zufuhr und Druckbeaufschlagung des einzuspritzenden Kraftstoffs,
Mittel (7) zur Temperierung der Brennkammer (1),
Mittel (10) zum Befüllen der Brennkammer (1) mit unter Druck stehender Verbrennungsluft, und eine ventilgesteuerte (15) Abgasleitung (12) zum Entlüften der Brennkammer (1),
und **durch** Mittel (14) zur Aufnahme entweder des zeitlichen Druckverlaufs oder des zeitlichen Lichtverlaufs in der Brennkammer (1) während der Zündung und Verbrennung von eingespritztem Kraftstoff.

2. Apparatur nach Anspruch 1, **gekennzeichnet durch** einen automatisierten Probengeber (3), mit dem die Mittel (4) zur Zufuhr des Kraftstoffs verbunden sind.

3. Apparatur nach Anspruch 2, wobei der automatische Probengeber (3) eine Mehrzahl von wahlweise zugreifbaren Probengefäßen enthält.

4. Apparatur nach einem der Ansprüche 1 bis 3, wobei die Einspritzdüse durch einen Piezo- oder Magnet-Kraftstoffinjektor (2) gebildet ist.

5. Apparatur nach einem der Ansprüche 1 bis 4, wobei die Einspritzdüse (2) über einen sie umgebenden Kühlmantel temperaturgesteuert wird, der von einer thermostatischen Flüssigkeit durchflossen ist.

6. Apparatur nach Anspruch 5, wobei der Kühlmantel der Einspritzdüse selbstragend ausgebildet und abnehmbar an der Brennkammer (1) angeordnet ist.

7. Apparatur nach einem der Ansprüche 1 bis 6, wobei die Kraftstoffleitung (16) von den Mitteln (4) zur Zufuhr und Druckbeaufschlagung des Kraftstoffs bis zur Einspritzdüse (2) temperaturgesteuert wird.

8. Apparatur nach einem der Ansprüche 1 bis 7, wobei die Mittel (4) zur Zufuhr und Druckbeaufschlagung des Kraftstoffs eine als Kolbenverdichterpumpe ausgebildete Hochdruckpumpe mit integrierter Druckregelung umfassen.

9. Apparatur nach einem der Ansprüche 1 bis 8, wobei die Mittel (7, 8) zum Temperieren der Brennkammer (1) eine dem Brennkammermantel gleichmäßig umgebende elektrische Widerstandsheizung (7) und einem Temperaturregler umfassen.

10. Apparatur nach einem der Ansprüche 1 bis 9, wobei die Mittel zur Aufnahme des Druckverlaufs in der Brennkammer (1) einen Quarz-Drucksensor (14) umfassen.

11. Apparatur nach einem der Ansprüche 1 bis 9, wobei die Mittel zur Aufnahme des Lichtverlaufs in der Brennkammer eine optische Sonde umfassen.

12. Apparatur nach einem der Ansprüche 1 bis 11, deren Temperatursteuervorgänge und deren Betriebsablaufsteuerung rechnergesteuert erfolgen.

13. Verfahren zur Oktanzahlbestimmung von Ottokraftstoffen unter Verwendung einer Apparatur nach einem der Ansprüche 1 bis 12, mit folgenden Schritten:
a) Auswahl einer Kraftstoffprobe,
b) Spülen der kraftstoffführenden Bauteile mit der gewählten Kraftstoffprobe,
c) Fördern der Kraftstoffprobe und Druckaufbau der Kraftstoffprobe bis auf den gewünschten Einspritzdruck,
d) Einstellen eines definierten Luftdrucks in der Brennkammer und Aufheizen der Verbrennungsluft in der Brennkammer auf eine definierte Temperatur,
e) Auslösen des Einspritzvorgangs und gegebenenfalls der Fremdzündung in der Brennkammer,
f) Aufnahme des zeitlichen Druckverlaufs oder des zeitlichen Lichtverlaufs in der Brennkammer, und Bestimmung des Zündverzugs und der Oktanzahl aus dem Druckverlauf oder dem Lichtverlauf,
g) Ausblasen des Abgases aus der Brennkammer.

14. Verfahren nach Anspruch 13, wobei die Schritte c) bis g) ein- oder mehrfach wiederholt werden.
